(19) Europäisches Patentamt
European Patent Office
Office européen des brevets

(11) EP 1 970 061 A1

(12) EUROPEAN PATENT APPLICATION
published in accordance with Art. 158(3) EPC

(43) Date of publication:
**17.09.2008 Bulletin 2008/38**

(21) Application number: **05857676.0**

(22) Date of filing: **28.12.2005**

(51) Int Cl.:
***A61K 31/405*** *(2006.01)* ***A61K 31/215*** *(2006.01)*
***A61K 31/13*** *(2006.01)* ***A61K 31/351*** *(2006.01)*
***A61K 31/7056*** *(2006.01)* ***A61K 31/196*** *(2006.01)*
***A61P 31/12*** *(2006.01)*

(86) International application number:
**PCT/RU2005/000677**

(87) International publication number:
**WO 2007/075102 (05.07.2007 Gazette 2007/27)**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **Zakrytoe Aktsionernoe Obschestvo "Masterklon"**
**Moscow 127473 (RU)**

(72) Inventor: **LENEVA, Irina Anatolievna**
**Moscow 123242 (RU)**

(74) Representative: **HOFFMANN EITLE**
**Patent- und Rechtsanwälte**
**Arabellastrasse 4**
**81925 München (DE)**

# (54) MEDICINAL AGENT FOR TREATING VIRAL INFECTIONS

(57) The invention relates to medicine, in particular to searching and developing novel medicinal agents for treating and preventing viral infections, mainly influenza viruses. The aim of said invention is to develop more efficient and low toxic medicinal agents by combining arbidol and the analogs thereof with a preparation exhibiting another mechanism of action. Said combinations enhances the efficiency of the preparation small doses and makes it possible to reduce the probability of side effects and appearance of virus resistant strain by reducing the dose. The result is attained by the combined use of arbidol and the analogies thereof with at least one type of preparation selected from the following group of antiviral preparations: ribavirin, zanamivirin, oseltavirin, peramirin, amantadin or remantadin.



Printed by Jouve, 75001 PARIS (FR)

## Description

The invention relates to medicine, in particular to searching for and developing novel medicinal agents for treating and preventing viral infections, mainly due to influenza viruses.

A large number of antiviral preparations are known at the present moment. Preparations of the adamantane series: amantadin and remantadin, relate to the first generation of such preparations. The antiviral effect of these preparations is due to the fact that the aforesaid preparations block the ionic channels formed by the transmembrane region of the M2 viral protein. The amantadin and remantadin molecules, corresponding in respect to size to the diameter of these ionic channels, inhibit a transfer of protons, thus increasing the pH inside the endosemes. As a result of this, conditions are created inside the virion that prevent the yield of ribonucleoprotein and the transcription of a viral genome. The virus reproduction suppression is the consequence of such a process. However, preparations of the adamantane series are efficient only in respect to influenza A viruses. Furthermore, their use is limited by the presence of serious side effects and the rapid appearance and spread of strains resistant thereto.

Ribavirin - a purine analog of nucleosides, is sufficiently effective against influenza A and B viruses, but should be used with care by patients with previous anemia, diabetes or disease of the coronary arteries. Furthermore, ribavirin has a teratogenic effect.

Neuraminidase inhibitors: zanamivir, oseltamivir and peramivir, relate to preparations of the second generation. Neuraminidase is one of the main enzymes participating in the replication of influenza A and B viruses. Upon its inhibition, the release of new viral particles from infected cells and the further spread of the virus in the organism are difficult. The relatively often (about 10%) occurring side effects in the form of nausea and vomiting upon the administration of oseltamivir, the occurrence of irritation of the nasopharynx upon the administration of zanamivir are drawbacks of these medicines. Furthermore, the latter are quite expensive. The use of neuraminidase inhibitors at present is even more complex in view of the fact that recently, in February 2005, an influenza A virus, H5N1, stable in respect to oseltamivir was isolated (Nature, 2005, vol. 437, No. 20). The possibility for transfer of this virus from person to person was confirmed.

The most close analogue of this invention is 1-methyl-2-phenylthiomethyl-3-carbethoxy-4-dimethylaminomethyl-5-oxy-6-bromindol hydrochloride (arbidol), intended for the treatment and prophylaxis of influenza A and B in adults and children (Patent RU No. 2008004). The mechanism of the antiviral action of this preparation has not been exactly established, but it is presumed that arbidol prevents the fusion of lipid shell of a virus and cellular membranes. But this preparation is mainly effective at the initial stages of the disease.

Taking the aforesaid into account, the search for new preparations for treatment of viral infections is still very actual.

The technical result of the instant invention is the creation of more effective and less toxic medicinal agents as a result of a combination of arbidol or structural analogs thereof that have proven antiviral activity with an antiviral preparation that has another mechanism of action. Such a combination enhances the efficacy of low doses of the preparations, and also, due to a reduction of the dose, makes it possible to reduce the probability of manifestation of side effects and the occurrence of resistant strains of the virus.

The aforesaid result is achieved upon the combined use of arbidol or analogues thereof and at least one preparation selected from the following groups of antiviral preparations: ribavirin; inhibitors of viral neuraminidase, preferably zanamivir, oseltamivir or peramivir; $M_2$ blockers channels, preferably amantadin or remantadin.

In the claimed invention arbidol (or analogues thereof) together with zanamivir, peramivir, oseltamivir, amantadin, remantadin or ribavirin manifest a significantly higher activity, as a result of which it became possible to reduce the content thereof in the preparation with the achievement of the same or even stronger therapeutic effect.

Active analogues of arbidol, which have antiviral properties, may be described with the aid of formula I:

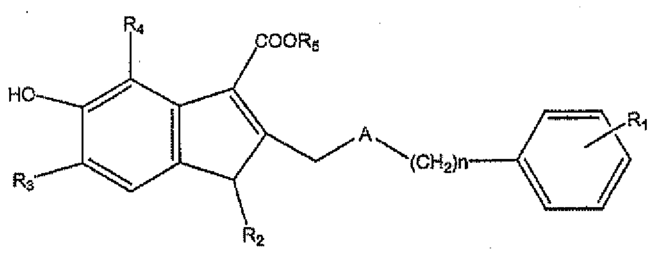,

wherein

$R_1$ is alkyl with the number of carbon atoms from 1 to 5; oxyalkyl, chlorine;

$R_2$ is alkyl with the number of carbon atoms from 1 to 5, phenyl, substituted phenyl, where alkyl, oxyalkyl, chlorine, bromine may be used as the substituent;
$R_3$ is H or Br;
$R_4$ is H, $CH_2N(CH_3)_2$;
$R_5$ is H, $COOC_2H_5$;
n = 0-2;
A is S, $SO_2$, SO.

Analogues of arbidol, corresponding to formula I, including the synthesis and physicochemical properties thereof, are described in literature (Pharm. Chem. Journal, No. 1, 1995, pages 51-53; Pharm. Chem. Journal, No. 2, 1995, pages 5-8; Pharm. Chem. Journal, No. 1, 1998, pages 565-569). The most active compound - 1-methyl-2-phenylthiomethyl-3-carbethoxy-5-oxy-6-bromindol sulfon was detected among the aforesaid analogues, this compound having the following structural formula:

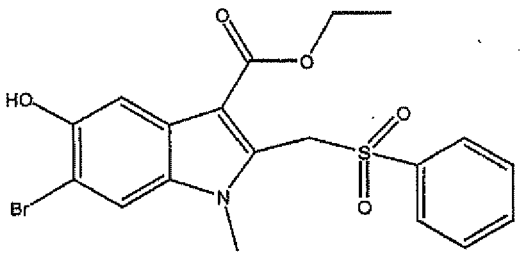

It was unexpectedly detected as a result of the study that 1-methyl-2-phenylthiomethyl-3-carbethoxy-4-dimethylaminomethyl-5-oxy-6-bromindol sulfon has significantly less toxicity than arbidol, while the achieved antiviral effect is comparable with and even exceeds the same in arbidol and other studied analogues of arbidol. This compound may be used both independently for the treatment of viral infections and in combination with preparations from the aforesaid groups, which significantly enhances the effectiveness of action.

In the most general variant, the instant invention discloses medicinal agents, wherein they comprise an effective amount of a compound of general formula I as one of the components, and as another - an effective amount of an antiviral agent with another mechanism of action, which belongs to one of the aforementioned groups of medicinal agents, in particular: preparation of the adamantane series, preferably - amantadin or remantadin; neuraminidase inhibitors, preferably - zanamivir, oseltamivir or peramivir; or remantadin.

It is most preferable that such compositions comprise 1-methyl-2-phenylthiomethyl-3-carboxy-4-dimethylaminomethyl-5-oxy-6-bromindol hydrochloride (arbidol) or 1-methyl-2-phenylthiomethyl-3-carbethoxy-4-dimethylaminomethyl-5-oxy-6-bromindol sulfon (sulfon) or pharmaceutically acceptable salts of these compounds in combination with oseltamivir.

The aforesaid combinations may be used as different medicinal forms, for example, as a solid medicinal form, preferably as tablets or capsules, and also as a solution for injection. Wherein, pharmaceutical carriers, accepted in pharmaceutical chemistry for the preparation of means for the delivery of an active component into the organism of a patient, may be used. Furthermore, the preparations may additionally contain fillers, binders, target additives, etc. (for example, as indicated in the 2nd edition, London: The Pharmaceutical Press; 1994). The target additives include complex-forming agents, such as EDTA, ascorbic acid and other antioxidants, hydrocarbons, such as dextrin, hydroxy alkyl cellulose, hydroxy alkyl methyl cellulose, stearic acid, etc. In addition to the ingredients mentioned above, the preparations may also include other traditional additives, for example, taste, aromatic, etc.

Combinations of medicinal preparations, in accordance with the instant invention, are also suitable for the preparation of pharmaceutical preparations with controlled release in which the release of the active ingredients is controlled and regulated in order to reduce the frequency of administration of the medicinal dose and improve the pharmacokinetic profile.

The effective dose of the active component depends on whether the compound is used for the prophylaxis (lower doses) or the treatment of a viral infection. Furthermore, the effective dose may also depend on the weight, age, sex of the patient and the presence of accompanying diseases.

The invention is illustrated by the following examples.

Example 1

The action of arbidol and analogues thereof in combination with inhibitors of viral neuraminidase on the reproduction of the influenza A/Singapore/1/57 virus in the MDCK cell culture.

A study of the antiviral activity of the preparations (in all of the examples) was carried out on a MDCK cell culture by the method of immunoenzymatic analysis. The percent of inhibition of viral reproduction by the studied preparation was determined by the formula:

$$\% \text{ of inhibition} = 100\text{-}(OD_{490} \text{ experiment} - OD_{490} \text{ cellular control}) / (OD_{490} \text{ viral control in the absence of the compound} - OD_{490} \text{ cellular control}).$$

Where $OD_{490}$ is the optical density at a corresponding wavelength.

The designations of the studied compounds: A - arbidol, B - 1-methyl-2-(4-methylphenylsulfomethyl-3-carbethoxy-4-dimethylaminomethyl-5-oxy-6-bromindol, C-1-methyl-2-(4-oxyethyl-phenylsulfomethyl)-3-carbethoxy-4-dimethylaminomethyl-5-oxy-6-bromindol. The percents of inhibition corresponding to these compounds (A, B, C) are presented in Table No. 1 through a comma.

Table No. 1

| A or B (µg/ml) | Percent of inhibition of OD 490 (Time of adding the preparation - 2 hours after infection) | | | | | |
|---|---|---|---|---|---|---|
| | Zanamivir (µg) | | | | | |
| | 0 | 0.3 | 1 | 3.3 | 10 | 33 |
| 0 | no inhib. | 27,15 | 29,15 | 36,20 | 43,22 | 49,28 |
| 0.3 | no inhib. | 20,10 | 30,15 | 37,21 | 48,30 | 60,40 |
| 1 | no inhib. | 33,21 | 40,26 | 46,30 | 100,85 | 100,83 |
| 3 | no inhib. | 60,40 | 58,45 | 93,80 | 100,90 | 100,87 |
| 5 | no inhib. | 69,52 | 85,80 | 88,75 | 100,96 | 100,95 |
| 10 | 22,10 | 71,50 | 85,75 | 98,90 | 100,100 | 100,100 |
| | Oseltamivir (µg) | | | | | |
| A or C (µg/ml) | 0 | 0.4 | 1.2 | 4.1 | 12.3 | 41 |
| 0 | - | 36,18 | 39,18 | 37,20 | 48,34 | 52,36 |
| 0.3 | no inhib. | 35,15 | 40,16 | 37,18 | 47,35 | 55,40 |
| 1 | no inhib. | 48,30 | 59,31 | 56,41 | 100,88 | 100,90 |
| 3 | no inhib. | 62,35 | 58,37 | 90,60 | 100,91 | 100,100 |
| 5 | no inhib. | 79,50 | 84,40 | 100,96 | 98,96 | 100,100 |
| 10 | 20,10 | 70,46 | 85,65 | 100,95 | 100,95 | 100,98 |

It is evident from the presented table that arbidol, added to the cells 2 hours after the infection thereof, has insignificant action on reproduction of the virus. Similarly, both inhibitors of viral neuraminidase, taken separately, make it possible to achieve only about 50% inhibition. However, the joint use of arbidol or analogues thereof with neuraminidase inhibitors 2 hours after infection makes it possible to significantly enhance the effect of both preparations. The combination of 3 µg/ml of arbidol and 3.3 mg of zanamivir (oseltamivir) made it possible to achieve 90% inhibition of the virus, while a ten times greater amount of zanamivir or oseltamivir without the addition of arbidol suppresses reproduction by only 50%.

When 10-12 mg of zanamivir or oseltamivir are used, only 1 µg of arbidol is sufficient to achieve 100% suppression of virus reproduction.

Example 2

The action of arbidol (A) and the analog thereof - 1-methyl-2-(n-methylphenylsulfomethyl)-3-carbethoxy-4-dimethylaminomethyl-5-oxy-6-bromindol (D) in combination with blockers of $M_2$-channels on the reproduction of the influenza A/Singapore/1/57 virus in a culture of MDCK cells.

The percents of inhibition corresponding to the indicated compounds (A, D) are presented in Table No. 2 through a comma.

Table No. 2

| A or D (μg/ml) | Percent of inhibition of OD 490 (time for adding the preparation - 30 min prior to infection) | | | | | | | | | |
|---|---|---|---|---|---|---|---|---|---|---|
| | Amantadin (μg/ml) | | | | | Remantadin (μg/ml) | | | | |
| | 0 | 0.3 | 1 | 5 | 10 | 0 | 0.3 | 1 | 5 | 10 |
| 0 | no inhib. | 22,6 | 28,10 | 50,30 | 56,38 | no inhib. | 25,10 | 31,12 | 56,41 | 59,40 |
| 1 | 30,15 | 30,17 | 30,18 | 52,29 | 59,45 | 30,12 | 31,18 | 33,20 | 50,36 | 59,42 |
| 5 | 38,24 | 60,38 | 68,41 | 74,53 | 81,70 | 38,20 | 63,48 | 71,44 | 74,50 | 82,73 |
| 10 | 67,40 | 77,57 | 81,70 | 82,75 | 85,80 | 67,54 | 78,69 | 80,69 | 82,70 | 86,75 |

A combination of arbidol with blockers of M2-channels also makes it possible to achieve a higher antiviral effect than upon the separate use of the same preparations, but in this case higher concentrations of arbidol are necessary than in Example No. 1. In experiments with amantadin, as is evident from Table 2, the highest synergistic effect is observed with a concentration of arbidol beginning with 5 μg/ml and concentrations of amantadin 0.3-5 μg/ml. Similar data were also obtained with the use of remantadin.

Example 3

The action of arbidol in combination with ribavirin on the reproduction of influenza A/Singapore/1/57 virus in a culture of MDCK cells.

Table No. 3

| Arbidol (μg/ml) | Ribavirin (μg/ml) | | | | |
|---|---|---|---|---|---|
| | 0 | 1 | 3 | 10 | 15 |
| 0 | - | 5 | 25 | 30 | 38 |
| 3 | - | 19 | 30 | 52 | 54 |
| 10 | 23 | 26 | 35 | 85 | 82 |
| 15 | 40 | 43 | 41 | 82 | 80 |
| 20 | 55 | 65 | 68 | 80 | 82 |

The addition of ribavirin to different concentrations of arbidol also increased the inhibition effect thereof, but the degree of increase turned out to be different for different concentrations. The combination 10 μg arbidol + 10 μg ribavirin turned out to be most favorable.

Example 4

The antiviral action of arbidol in combination with oseltamivir on the reproduction of avian influenza virus (H5N1) on an experimental model (white mice).

Animals: white mice having a weight from 15 to 20 g, obtained from a brooder of the Virusological Center of NIIM MO RF (the city Sergiev Posad).

The preparations were administered orally.

Assessment of the effectiveness of the preparations was carried out in respect to a reduction of the degree of

lethality of the mice.

Table 4

| Arbidol, mg/kg | Oseltamivir, mg/kg | Surviving mice, % |
|---|---|---|
| 30 | 2.5 | 100% |
| 30 | 1.0 | 85% |
| 30 | 0.25 | 70% |
| 0 | 2.5 | 60% |
| 30 | 0 | 65% |

Example 5

Determination of toxicity of 1-methyl-2-phenylthiomethyl-3-carbethoxy-5-oxy-6-bromindol sulfon as compared with arbidol.

Table No. 5

| Dose, mg/kg | 100 | 250 | 500 | 1000 | 3000 | 5000 | 7500 |
|---|---|---|---|---|---|---|---|
| Lethal effect upon administration to mice | | | | | | | |
| Arbidol | 0/6 | 0/6 | 1/6 | 3/6 | 6/6 | - | - |
| Sulfon | 0/6 | 0/6 | 0/6 | 0/6 | 2/6 | 4/6 | 6/6 |
| Lethal effect upon administration to rats | | | | | | | |
| Arbidol | 0/6 | 0/6 | 1/6 | 3/6 | 6/6 | - | - |
| Sulfon | 0/6 | 0/6 | 0/6 | 0/6 | 2/6 | 4/6 | 6/6 |

The administration of the preparations was carried out endogastrically, the period of observation of the animals was 14 days.

As is evident from the table, 1-methyl-2-phenylthiomethyl-3-carbethoxy-5-oxy-6-bromindol sulfon turned out to be significantly less toxic than arbidol. This is confirmed by the values of $LD_{50}$. In the case of arbidol this parameter was $860 \pm 50$ mg/kg, while for sulfon - $3600 \pm 180$ mg/kg in respect to mice, i.e., 4 times greater. The same parameter for rats is respectively - $1720 \pm 70$ mg/kg and $5350 \pm 250$ mg/kg, i.e., 3 times greater than with arbidol.

Example 6

Assessment of the antiviral activity of 1-methyl-2-phenylthiomethyl-3-carbethoxy-5-oxy-6-bromindol sulfon as compared with arbidol in the case of an influenza virus A/PR/8/34 in a culture of MDCK cells.

Table No. 7

| Compound | Percent of suppression of virus reproduction |
|---|---|
| Arbidol | 60 |
| Sulfon | 75 |

The efficacy of the preparation was studied on the example of an influenza virus in an MDCK cell culture by a standard method of immunoenzymatic analysis. The results showed that the efficacy of the new compound exceeds same for the known preparation arbidol.

Example 7

Assessment of the efficacy of 1-methyl-2-phenylthiomethyl-3-carbethoxy-5-oxy-6-bromindol sulfon as compared with arbidol in vivo.

Table No. 8

| Compound | Concentration | Percent of surviving mice |
|---|---|---|
| Prophylactic scheme | | |
| Arbidol | 15 | 80% |
| | 30 | 70% |
| Sulfon | 15 | 90% |
| | 100 | 60% |
| Therapeutic-prophylactic scheme | | |
| Arbidol | 30 | 80% |
| Sulfon | 30 | 70% |
| | 100 | 40% |
| Therapeutic scheme | | |
| Arbidol | 30 | 60% |
| Sulfon | 30 | 70% |
| | 100 | 50% |

A study of the antiviral activity, carried out on mice infected with the influenza virus H3N2, was carried out with the use of three schemes: prophylactic - the preparations were administered 24 hours before and then again 1 hour before infection; therapeutic-prophylactic - the preparations were administered 24 hours and 1 hour before infection and then 8 hours after infection; therapeutic - the preparations were administered 8 hours after infection and then each 8 hours for 14 days. The results show that the action of sulfon is comparable with the action of arbidol in the same concentrations.

1-Methyl-2-phenylthiomethyl-3-carbethoxy-5-oxy-6-bromindol sulfon may be used in different medicinal forms, including the case where it is used with pharmaceutical carriers that are accepted in pharmaceutical chemistry for the preparation of agents for the delivery of the active component into the organism of a patient.

## Claims

1. Use of 1-methyl-2-phenylthiomethyl-3-carbethoxy-5-oxy-6-bromindol sulfon for the production of a medicinal agent for treating or prophylaxis viral infections.

2. The use according to claim 1, **characterized in that** the virus is an influenza virus.

3. A pharmaceutical composition comprising 1-methyl-2-phenylthiomethyl-3-carbethoxy-5-oxy-6-bromindol sulfon in an effective amount and a pharmaceutically acceptable carrier.

4. A medicinal agent for treating or prophylaxis viral infections, **characterized in that** it comprises a compound of general formula I or pharmaceutically acceptable salts thereof and/or hydrates thereof:

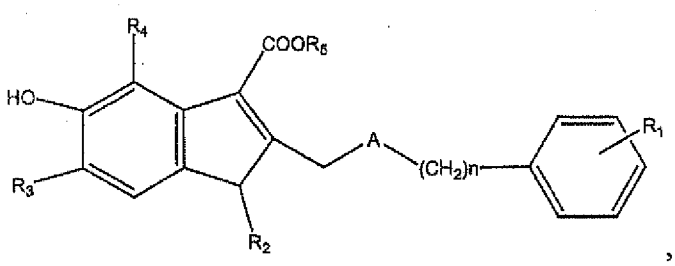

,

wherein

$R_1$ is alkyl with the number of carbon atoms from 1 to 5; oxyalkyl, chlorine;
$R_2$ is alkyl with the number of carbon atoms from 1 to 5, phenyl, substituted phenyl, where alkyl, oxyalkyl, chlorine, bromine may be used as the substituent;
$R_3$ is H or Br;
$R_4$ is H, $CH_2N(CH_3)_2$;
$R_5$ is H, $COOC_2H_5$;
n = 0-2;
A is S, $SO_2$, SO
and an inhibitors of viral neuraminidase in effective amounts.

**5.** The medicinal agent according to claim 4, **characterized in that** the virus is an influenza virus.

**6.** The medicinal agent according to claim 4, **characterized in that** the compound of general) formula I is 1-methyl-2-phenylthiomethyl-3-carbethoxy-4-dimethylaminomethyl-5-oxy-6-bromindol or 1-methyl-2-phenylthiomethyl-3-carbethoxy-5-oxy-6-bromindol sulfon or pharmaceutically acceptable salts thereof and/or hydrates thereof.

**7.** The medicinal agent according to claim 4, **characterized in that** the inhibitor of viral neuraminidase is selected from the group comprising zanamivir, oseltamivir and peramivir.

**8.** A medicinal agent for treating or prophylaxis viral infections, **characterized in that** it comprises a compound of formula I or pharmaceutically acceptable salts thereof and/or hydrates thereof and a blocker of $M_2$-channels in effective amounts.

**9.** The medicinal agent according to claim 8, **characterized in that** the $M_2$-channels blocker is selected from the group comprising amantadin, remantadin.

**10.** The medicinal agent according to claim 8, **characterized in that** the compound of general formula 1 is 1-methyl-2-phenylthiomethyl-3-carbethoxy-4-dimethylaminomethyl-5-oxy-6-bromindol or 1-methyl-2-phenylthiomethyl-3-carbethoxy-5-oxy-6-bromindol sulfon or pharmaceutically acceptable salts thereof and/or hydrates thereof.

**11.** A medicinal agent for treating or prophylaxis viral infections, **characterized in that** it comprise a compound of formula I or pharmaceutically acceptable salts thereof and ribavirin.

**12.** The medicinal agent according to claim 11, **characterized in that** the compound of general formula I is 1-methyl-2-phenylthlomethyl-3-carbethoxy-4-dimethylaminomethyl-5-oxy-6-bromindol or 1-methyl-2-phenylthiomethyl-3-carbethoxy-4-dimethylaminomethyl-5-oxy-6-bromindol sulfon or pharmaceutically acceptable salts thereof and/or hydrates thereof.

**13.** A medicinal agent for treating or prophylaxis viral infections, **characterized in that** it comprises a compound of formula I or pharmaceutically acceptable salts thereof and at least one of the preparations selected from the group comprising: amantadin, remantadin, zanamivir, oseltamivir, peramivir, ribavirin.

**14.** A method of treating or prophylaxis a viral infection, comprising administering to a mammal a therapeutically effective amount of the medicinal agent according to any of claims 1-13.

INTERNATIONAL SEARCH REPORT

International application No.
PCT/RU2005/000677

A. CLASSIFICATION OF SUBJECT MATTER **see sup. sheet**

According to International Patent Classification (IPC) or to both national classification and IPC

B. FIELDS SEARCHED

Minimum documentation searched (classification system followed by classification symbols)

A61K 31/00-31/7056, A61P 31/00-31/12

Documentation searched other than minimum documentation to the extent that such documents are included in the fields searched

Electronic data base consulted during the international search (name of data base and, where practicable, search terms used)

Esp@cenet, Enterez PubMed, JOPAL, EAPO, DEPATISnet, USPTO, PAJ, RUPATIS, ChemIDplus

C. DOCUMENTS CONSIDERED TO BE RELEVANT

| Category* | Citation of document, with indication, where appropriate, of the relevant passages | Relevant to claim No. |
|---|---|---|
| A | RU 2008004 C1 (TSENTR PO KHIMII LEKARSTVENNYKH SREDSTV-VNIKHFI), 28.02.94, left column, lines 1-21, 29-32, 48-64, rigth column, lines 53-67 | 1-14 |
| A | RU 2262350 C2 (GOSUDARSTVENNOE UCHREZHDENIE "MOSKOVSKY NAUCHNO-ISSLEDOVATELSKY INSTITUT EPIDEMIOLOGII I MIKROBIOLOGII IM. G.N. GABRICHEVSKOGO MINISTERSTVA ZDRAVOOKHRANENIYA ROSSIISKOI FEDERATSII"), 20.10.2005, page 2, page 7, lines 46-50, page 9, lines 16, 17, 25, page 12, lines 6, 8, 9 | 1-14 |
| A | O.S. Anisimova et al. "Issledovanie metabolizma protivovirusnogo preparata arbidol metodami TSKH, VEZHKH i Mass-spektromii". Khimiko-farmatsevtichesky jurnal, Moscow, Meditsina, 1995, volume 29, No 2, pages 5-8 | 1-14 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 28 August 2006 | 07 September 2006 |
| Name and mailing address of the ISA/ **RU** | Authorized officer |
| Facsimile No. | Telephone No. |

Form PCT/ISA/210 (second sheet) (April 2005)

INTERNATIONAL SEARCH REPORT

International application No.
PCT/RU2005/000677

C (Continuation). DOCUMENTS CONSIDERED TO BE RELEVANT

| Category* | Citation of document, with indication, where appropriate, of the relevant passages | Relevant to claim No. |
|---|---|---|
| A | S.A. Zotovaet al. "SINTEZ I PROTIVOVIRUSNAYA AKTIVNOST SULFIDOV INDOLA I BENZOFURANA", Khimiko-farmatsevtichesky jurnal. Moscow, Meditsina, 1995, volume 29, No 1, pages 51-53 | 1-14 |
| A | US 2004/0062801 A1 (JOAQUINA FAOUR et al.) 01.04.2004, the abstract page 1, paragraphs [0001], [0004], [0005], 0007] | 1-14 |
| A | Entsiklopediya lekarstv under editorship Ju. F Krylova, Moscow, RLS, 2000, pages 63, 791, 797, 799-800 | 1-14 |

Form PCT/ISA/210 (continuation of second sheet) (April 2005)

INTERNATIONAL SEARCH REPORT
Information on patent family members

International application No.
PCT/RU2005/000677

*A61K 31/405 (2006.01)*
*A61K 31/215 (2006.01)*
*A61K 31/13 (2006.01)*
*A61K 31/351 (2006.01)*
*A61K 31/7056 (2006.01)*
*A61K 31/196 (2006.01)*
*A61P 31/12 (2006.01)*

Form PCT/ISA/210 (patent family annex) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- RU 2008004


### Non-patent literature cited in the description


- *Nature,* 2005, vol. 437 (20
- *Pharm. Chem. Journal,* 1995, vol. 1, 51-53
- *Pharm. Chem. Journal,* 1995, vol. 2, 5-8
- *Pharm. Chem. Journal,* 1998, 565-569